# EUROPEAN PATENT SPECIFICATION

(11) **EP 0 741 371 B1**
(45) Date of publication and mention of the grant of the patent: **23.10.2002**
(21) Application number: 95201120.3
(22) Date of filing: 01.05.1995
(51) Int. Cl.: G06T 5/00, G06T 3/40

(54) **Radiation image reproducing method**
Verfahren zur Wiedergabe eines Strahlungsbildes
Méthode de reproduction d'image de rayonnement

(43) Date of publication of application: 06.11.1996
(73) Proprietor: AGFA-GEVAERT, 2640 Mortsel (BE)
(72) Inventor: Dhaenens, Frans, c/o Agfa-Gevaert N.V., B-2640 Mortsel (BE)

(56) References cited:
- EP-A- 0 146 728
- EP-A- 0 610 604
- US-A- 4 969 204
- US-A- 5 022 091

## Description

### 1. Field of the invention.

The present invention is in the field of digital radiography and more specifically relates to hard copy recording of radiation images.

### 2. Background of the invention

It is common practice in radiographic diagnosis to follow a patient's condition by comparing radiographic images relating to X-ray exposures of a patient taken at different points in time.

Especially in an intensive care department of a hospital such a comparative study is part of daily routine.

In classical radiography where an X-ray image of a patient is acquired by exposing a radiographic film to X-rays transmitted by a patient, such a comparative study is performed by placing old and new radiographic films pertaining to the same patient (and/or same examination) besides each other on a light box in a way that is optimal for comparison of the items on the radiographic images the radiologist is interested in.

Often however, old radiographic images are not easily retrievable. Films relating to older exposures may be not immediately available at the location where the radiologist wants to make the evaluation. The older images may for example be classified in a patient's medical file kept at another department than the radiology or the intensive care department, or they may have to be retrieved from a central hospital archive.

It is even possible that older radiographs are kept by the patient himself at his private premises or, in the worst case, that older radiographic images are lost.

Further, once old and new images are localized and gathered, they still have to be put on a lightbox in a way that is optimal for comparison of the items on the radiographic image the radiologist is interested in.

For this purpose a lightbox of adequately large dimensions must be available and the individual films have to be arranged on that lightbox in a way that enables easy comparison and evaluation by the radiologist.

This film handling is not very practical and may take a lot of time.

Recently in many radiographic applications the importance of digital acquisition techniques has increased.

In the field of digital radiography a wide variety of image acquisition techniques have been developed such as computerised tomography, nuclear magnetic resonance, ultrasound, detection of a radiation image by means of a CCD sensor or a video camera, radiographic film scanning etc.

Still another technique has been developed wherein a radiation image, for example X-rays transmitted by an object, is stored in a screen comprising a photostimulable phosphor such as one of the phosphors described in European patent application 503 702 published 16.09.92 and in US-A-5,340,661.

The technique for reading out the stored radiation image consists of scanning the screen with stimulating radiation, such as laser light of the appropriate wavelength, detecting the light emitted upon stimulation and converting the emitted light into an electric representation for example by means of a photomultiplier.

After read-out the residual image left on the photostimulable phosphor screen is erased so that the screen is again available for exposure.

This technique further comprises digitizing and processing said electric signal. Processing of the read out radiation image for the purpose of enhancing the contrast in the image has for example been described in European patent application 527.525, as well as in European patent application 610.604.

As described in the above applications, the digital signal representation of a radiation image is first decomposed into a sequence of detail images at multiple resolution levels and a residual image at a still lower resolution level. Then, the detail images are modified.

Next, the modified detail images and the residual image are recombined so as to form a processed image by application of a transformation to the modified detail images and the residual image.

The transformation is such that, if it would have been applied to the residual image and the unmodified detail images, then the original image or a close approximation thereof would result.

The processed digital signal representation can further be applied to a recorder for recording a hard copy for example on film. This hard-copy can be viewed on a lightbox for diagnostic purposes.

The electric image representation can also be applied to a monitor for display of the corresponding visual image.

Once the radiographic images are available in the form of a digital signal representation, they can be archived on a suitable storage medium such as an optical disc system.

Comparison of old and new radiation images has already been addressed in the prior art. For example the prior art document US 4,999,497 relates to a method wherein old and new images can be compared and wherein changes can be ascertained accurately.

According to the described image read out and reproducing method the image processing and/or read out conditions to be used for obtaining the second reproduced image are adjusted on the basis of the image information corresponding to the first reproduced image so that the second reproduced image has approximately the same gradation and the same sensitivity as the first image. Image read out is carried out by using the adjusted read-out condition and/or image processing is carried out by using the adjusted image processing conditions. Images are reproduced individually and are then compared.

Simultaneous display of more than one image has already been addressed in the prior art. For example, European patent application 567 176 relates to the simultaneous display of more than one new images, immediately after acquisition, as a mosaic type image on a display device.

According to the method disclosed in this application, a number of radiation images, each being represented by a digital signal representation, are displayed on a preview monitor. Reduced image signals are deduced from the digital signal representations of said radiation images, and a composed signal representing a mosaic type image is formed by means of said reduced image signals and by applying said composed signal to said display device.

A reduced image signal is obtained by the steps of (i) decomposing a digital signal representation of an image into a sequence of detail images at multiple resolution levels and a residual image at a resolution lower than the minimum of said multiple resolution levels, (ii) modifying pixel values of said detail images to yield pixel values of a set of modified detail images by means of at least one non-linear monotonically increasing odd mapping function with a slope that gradually decreases with increasing argument values and
(iii) computing a processed image by applying a reconstruction algorithm to the modified detail images up to an intermediate level of said multiple resolution levels and to the residual image, the reconstruction algorithm being such that when applied to the unmodified detail images and the residual image said original image or a close approximation thereof would be obtained.

The method of this application further provides that as a new reduced signal is deduced, said composed signal is amended by means of said new reduced signal so that at least one of the images of the mosaic type image is replaced by an image represented by said new reduced signal.

The invention disclosed in this application addresses a different problem, it does not relate to display of old (archived) images for comparison with other (e.g. new images). Further, the disclosed invention does not relate to reproduction.

Several publications such as FR 2.508.187 and EP 0 599 098 disclose reproduction of more than one differently processed version of a single image on a single photographic film. The different versions originate from a single image and are for example processed using different processing parameters, such as different edge enhancement, different tone scaling etc.

### 3. Objects of the invention.

It is a principal object of the present invention to provide a method for making comparative studies on radiographic images, for example on old and new images pertaining to the same patient.

It is a further object to provide a method of producing a patient folder in the form of a hard copy.

Further objects will become apparent from the description hereafter.

### 4. Statement of the invention

To achieve the above objects the invention provides a method of reproducing radiation images on a recording material comprising the steps of
(i)identifying radiation images that are to be reproduced,
(ii)extracting from a digital image representation of an image to be reproduced a reduced version representing a low resolution version of that image, characterised in that
(iii)images are identified in accordance with a rule, e.g. patient's name, day or radiology room,
(iv)for each of the identified radiation images a corresponding digital image representation is retrieved from an electronic archive,
(v)a composite image signal representing a composite image is formed comprising for each identified radiation image said low resolution image version,
(vi) said composite image signal is applied to a hard copy recorder to generate on a single recording material a composite image comprising a low resolution version of each of the identified radiation images.

A suitable recording material is a photographic material, for example a photographic film. However, alternatives may be envisaged such as a thermo-sensitive recording material etc.

The resolution of the low resolution version of the identified radiation images is lower than the original resolution of the radiation images. The low resolution however is preferably still adequate for evaluation by the radiologist.

Identification of radiographic images to be retrieved from archive can for example be performed by entering on a work station at least one rule the retrieved images must fulfil.

For example, on entry of a patient's name the images are retrieved that pertain to that patient.

Alternatively images may be retrieved that are taken in the same radiology room or on the same day, or that relate to the same examination type etc. Also logical combinations of several selection rules are possible.

The invention is particularly advantageous in case the images that are reproduced on a single radiographic film are old and new images pertaining to the same patient thus providing the radiologist with a so-called patient folder on a single radiographic film.

The technique of the present invention is advantageous over the prior art method wherein different hard copy images were arranged on a common light box, since it is far more convenient.

Hard copy images do not have to be gathered from several sources, nor does the radiologist have to arrange the hard copies on a light box in a way that permits comparison.

This technique is also advantageous when compared with display of images on a monitor because of the higher resolution and the superior grey value reproduction ability of hard copy recorders such as laser recorders.

Indeed, although the resolution of the individual images that are together reproduced on a photographic film will be lower than the resolution of the raw image that is provided by the image acquisition device, when not too many images are reproduced on a single film, the resolution of the low resolution image versions still remains adequate to permit the radiologist to make a diagnosis. This is a great advantage compared to display of images on a monitor. Monitors typically have a resolution of 1000x1000 pixels, even high resolution monitors typically do not have a resolution higher than 2000x2000 pixels, and these high resolution images are very expensive. Commonly available laser printers on the other hand are able to provide a resolution of 4000x5000 pixels. Laser printers having a higher resolution, even extending to 8000x10.000 pixels are expected for the near future.

Further, the number of grey values that can be reproduced by a laser printer is typically 2⁸, whereas monitors are only able to reproduce 2⁷ grey levels.

The reproducibility of images recorded by means of hard copy recorders such as laser printers is better than that provided by monitors. Images displayed on different monitors are hard to compare because identical setting of monitors can hardly be achieved.

In one embodiment said low resolution representation is obtained by subsampling of pixel values of said digital signal representation.

In another specific embodiment a radiation image has been stored in said electronic archive in the form of a multi-resolution signal representation that has been obtained by decomposing a digital image representation of an image into a sequence of detail images at multiple resolution levels and a residual image at a resolution level still lower than the lowest of said multiple resolution levels, the decomposition being characterised in that a reconstruction algorithm exists that, when it is applied to all detail images and the residual image, renders the original image or a close approximation thereof.

The low resolution image version is then obtained by applying a reconstruction algorithm to the detail images up to an intermediate of said resolution levels and to the residual image, the reconstruction algorithm being such that when it is applied to all detail images and the residual image (without any of them having been subjected to modification) that then the original image or a close approximation thereof would be obtained.

The intermediate resolution level itself is determined by the number of radiation images that is envisaged to be reproduced on a single radiographic film as well as by the resolution of the recording device and the film format.

In a preferred embodiment the low resolution version is a processed low resolution version obtained by applying at least one modifying function to at least some of the detail images.

The modifying function is preferably a monotonically increasing odd mapping function with a slope that gradually decreases with increasing argument values.

This kind of function provides contrast improvement of a digital image over the whole range of signal levels without enlarging the dynamic range in a system for reproducing or displaying an image read-out of a photostimulable phosphor screen.

This kind of processing further provides that independent of the exposure conditions, the optical density in certain parts of the image is constant for all processed and reproduced images. For example, the optical density in certain parts of the lungs is constant for all images independent of the processing conditions that were applied.

In a specific embodiment of the invention the original image is decomposed into a so-called pyramidal sequence of detail images, i.e. successively formed detail images in the set of multi-resolution detail images have a reduced number of pixels.

For example, the multi-resolution representation after decomposition may have a pyramidal structure such that the resolution level of the detail images differs by a factor of two, and the detail images at each resolution level are calculated by filtering the original image with filters having decreasing bandwidth.

The used filter preferably has a two-dimensional Gaussian distribution.

This procedure can be implemented as described below with reference to the accompanying drawings.

In a specific embodiment the workstation is connected to a so-called RIS (radiology information system) or HIS (hospital information system). These systems are data base systems wherein radiographic images are stored in an electronic form.

Upon identification, images can be retrieved from the HIS or RIS systems and can be subjected to the above-described decomposition and partial reconstruction and subsequently to the composition of the composed image that is reproduced by the recorder.

### 5. Brief description of the drawings

Particular aspects of the present invention as well as preferred embodiments thereof will be explained by means of the corresponding drawings wherein
Fig. 1 generally shows a system in which the method of the present invention can be applied,
Fig. 2 illustrates read-out of a radiation image that has been stored in a photostimulable phosphor screen,
Figure 3 is a plot of a specific modifying function that can be used in a method of the present invention,
Fig. 4 illustrates an embodiment of a decomposition process,
Figure 5 illustrates an embodiment of a reconstruction algorithm,

### 6. Detailed description

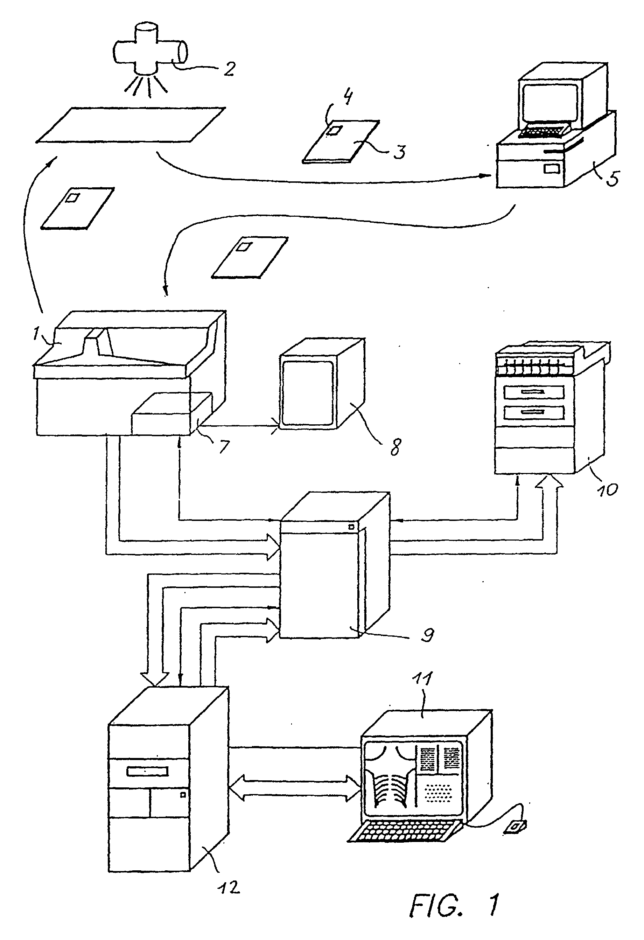

Figure 1 generally shows an apparatus in which the method of the invention can be applied.

The system comprises an exposure unit with an X-ray source 2 for exposing a photostimulable phosphor screen to X-rays transmitted by a patient, an identification station (5) for identifying a cassette conveying a photostimulable phosphor screen, a read-out (1) and processing (7) station, a preview monitor (8), a workstation (11, 12) and a hard copy recorder (10) with associated buffer (9).

A radiation image of an object was recorded on a photo-stimulable phosphor screen by exposing said screen to X-rays emitted by an X-ray source (2) and transmitted through the object (not shown). The stimulable phosphor screen was conveyed in a cassette (3) provided with an electrically erasable programmable read only memory (EEPROM) (4). In the identification station (5) various kinds of data, for example patient identification data (name, date of birth) and data relating to the exposure and/or to the signal processing were written into the EEPROM.

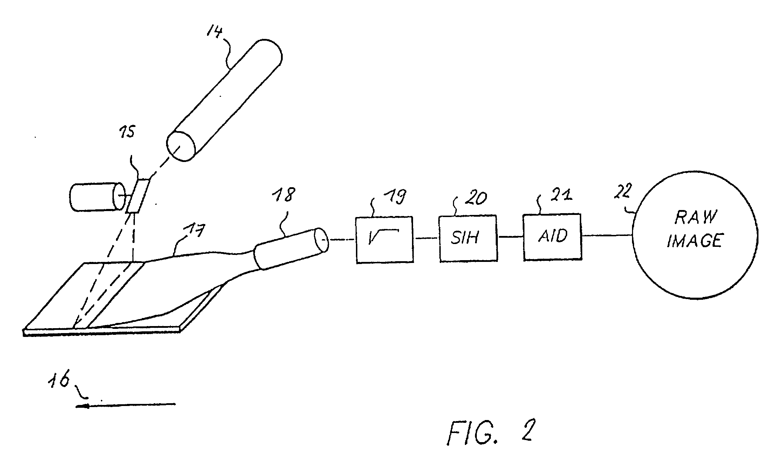

The read-out operation is shown in figure 2.

In the radiation image read-out station 1 the image stored in the photo-stimulable phosphor screen was read-out by scanning the phosphor screen with stimulating rays emitted by a laser (14). The stimulating rays were deflected into the main scanning direction by means of galvanometric deflection (15). The subscanning was performed by transporting the phosphor screen in the subscanning direction indicated by arrow (16). The stimulated emission was directed onto a photomultiplier (18) for conversion into an electrical image representation. Additionally the information stored in the EEPROM was read.

The output signal of the photomultiplier was converted by a square root amplifier (19), and next the signal was sampled by a sample and hold device (20) and quantised by an A/D converter (21). This quantised image signal, called the raw image signal (22), was sent to the image processing module of the read-out apparatus (figure 1, numeral 7) where it was subjected to a decomposition into a multi-resolution image representation. The decomposed image was also sent from the image processor to the image workstation (11, 12) where it was temporarily stored on a hard disc.
In processing module (7) the decomposed image signal was further processed taking into account the processing parameters associated with the data read out from the EEPROM (4) on the cassette conveying the photo-stimulable phosphor screen.

In the decomposition process the raw image signal (22) was decomposed into a sequence of detail images b₀, b₁, ...b_{L-1} which represent the amount of detail present in the raw image at multiple resolution levels, from fine to coarse. After the last decomposition step a residual image g_{L} was left.

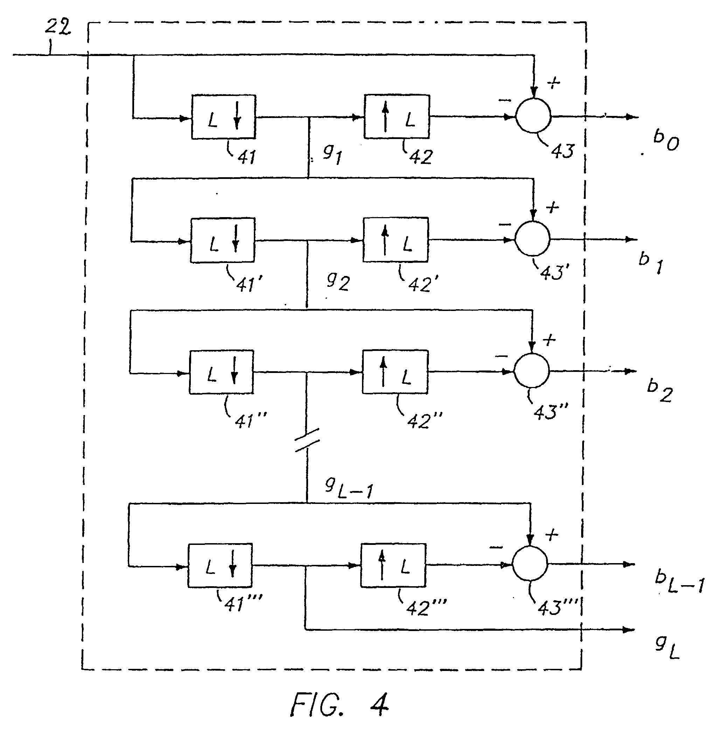

One embodiment of the decomposition process is illustrated in Fig. 4. Other embodiments that are applicable are described in European patent application EP-A-527 525.

The raw image (22) is filtered by a low pass filter (41), and subsampled by a factor of two, which is implemented by computing the resulting low resolution approximation image g₁ only at every other pixel position of every alternate row.

A detail image b₀ at the finest resolution level is obtained by interpolating the low resolution approximation image g₁ with doubling of the number of rows and columns, and pixelwise subtracting the interpolated image from the raw image.

The interpolation is effectuated by an interpolator (42), which inserts a column of zero values every other column and a row of zero values every other row respectively, and next convolves the extended image with a low pass filter. The subtraction is done by an adder (43).

The same process is repeated on the low resolution approximation g₁ instead of the raw image, yielding an approximation of still lower resolution g₂ and a detail image b₁.

A sequence of detail images bᵢ, i=0...L-1 and a residual low resolution approximation g_{L} are obtained by iterating the above process L times.

The finest detail image b₀ has the same size as the raw image.

The next coarser detail image b₁ has only half as many rows and columns as the first detail image b₀. At each step of the iteration the maximal spatial frequency of the resulting image is only half that of the previous finer detail image, and also the number of columns and rows is halved, in accordance with the Nyquist criterion.

After the last iteration a residual image g_{L} is left which can be considered to be a very low resolution approximation of the raw image. In the extreme case it consists of only one pixel which represents the average value of the raw image.

These detail images and the residual image are then sent to the work station (11, 12) for archival and later processing on the one hand and they are subjected to on-line processing in processing module (7) of the read out apparatus (1) on the other hand.

The method of the present invention is then applied to the detail images archived in the workstation.

Images to be reproduced on a single hard copy material were identified by entering specifics on these images, for example name of patient, data of examination etc. on the workstation.

Then, the identified images were retrieved from memory.

Then, a low resolution image was computed by applying a reconstruction algorithm to the retrieved residual image and to the retrieved detail images up to an intermediate of the multiple resolution levels of the detail images, the reconstruction algorithm being such that if it were applied to the unmodified detail images and the residual image, then the raw image or a close approximation thereof would result.

The intermediate resolution level is selected so that the retrieved images can all be reproduced on a single hard copy material.

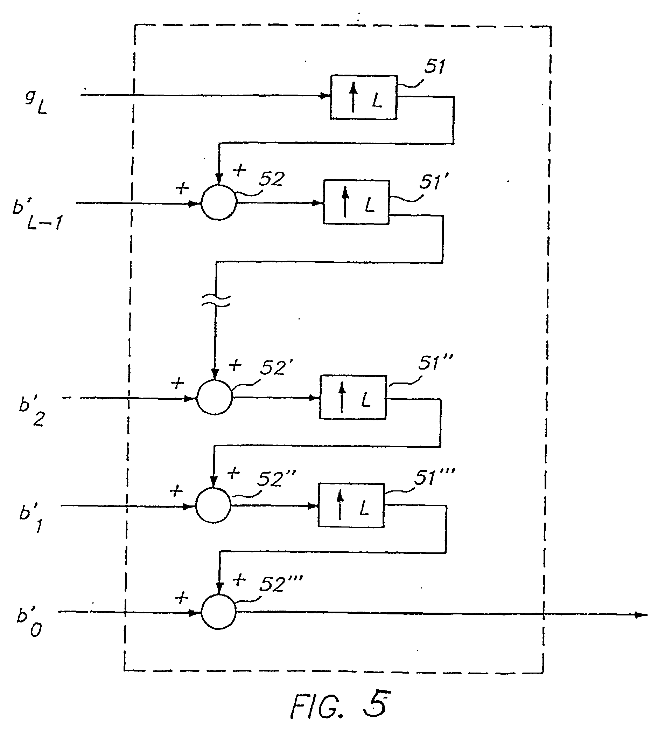

One embodiment of such a reconstruction algorithm is illustrated in figure 5, other embodiments can be found in the above mentioned European application EP-A-527 525.

In the reconstruction process, the residual image g_{L} is first interpolated by means of an interpolator (51) to twice its original size and the interpolated image is then pixelwise added to the detail image of the coarsest level b'_{L-1}, using an adder (52).

The resulting image is interpolated and added to the next finer detail image. If this process is iterated L times using the unmodified detail images b_{L-1} ... b₀, then an image equal to the raw image will result. If, at the other hand the detail images are modified before reconstruction, then a processed image, for example a contrast enhanced image will result. The interpolators are identical to those used in the decomposition section.

The low resolution image version that will be used for the composition of the composed image (or mosaic type image) to be recorded on a single photographic film then consists of an image generated during the reconstruction process being limited up to some intermediate resolution level which is lower than the original resolution, as explained higher.

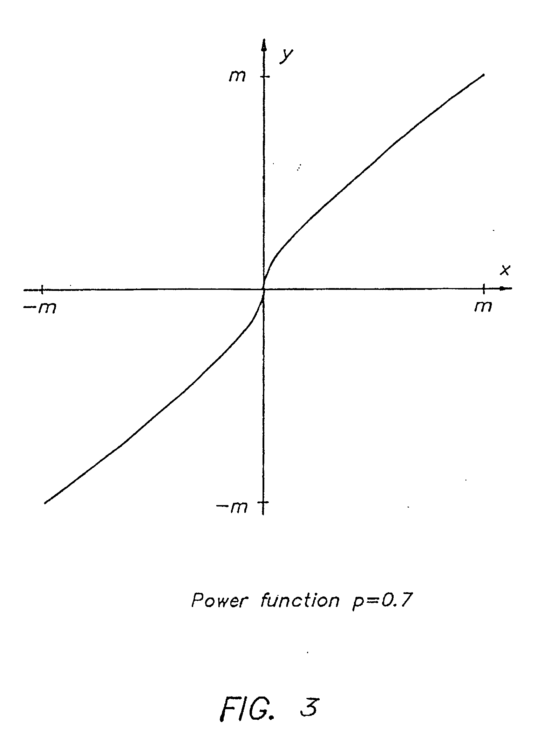

It is also possible to apply signal processing to the pixel values of the retrieved detail images.
For example, for the purpose of contrast enhancement, the pixel values of the detail images can be modified to yield pixel values of modified detail images . Preferably the modification is performed by applying to the pixel values of the detail images (or to pixel values of some of the detail images ) at least one non-linear monotonically increasing odd mapping function with a slope that gradually decreases with increasing argument values. Figure 3 is a plot of a specific modifying function that can be used in a method of the present invention.

The above described embodiment including modification of the detail images is advantageous in that the reconstructed images that are used for composition of the composed image have an enhanced image quality.

Finally, by means of the reconstructed image signals a composite image signal is generated and the composite signal is applied to a laser recorder for reproduction of a composite image comprising a low resolution version of each of the identified images.

An example of a laser recorder capable of accepting image signals in either analogue or digital form is the laser printer marketed by Agfa-Gevaert N.V. under the registered trade mark MATRIX COMPACT L.

This laser printer is suitable for recording the radiographic images generated by a digital radiographic system, or generated by other digital diagnostic techniques such as CT, MR and DSA.

Films suitable for being used in such laser printers are e.g. the photographic films marketed by Agfa-Gevaert N.V. under the registered trade marks SCOPIX LT, available in the following film formats : 8 x 10 inch, 14 x 11 inch or 14 x 17 inch.

Another example of a suitable laser printer is the MATRIX LR 3000 printer.

## Claims

1. A method of reproducing radiation images on a recording material comprising the steps of
(i)identifying radiation images that are to be reproduced,
(ii)extracting from a digital image representation of an image to be reproduced a reduced version representing a low resolution version of that image, **characterised in that**
(iii)images are identified in accordance with a rule, e.g. patient's name, day or radiology room,
(iv)for each of the identified radiation images a corresponding digital image representation is retrieved from an electronic archive,
(v)a composite image signal representing a composite image is formed comprising for each identified radiation image said low resolution image version,
(vi) said composite image signal is applied to a hard copy recorder to generate on a single recording material a composite image comprising a low resolution version of each of the identified radiation images.

2. A method according to claim 1 wherein
(i) a radiation image has been stored in said electronic archive in the form of a multi-resolution image representation obtained by applying to the digital image representation of said image a decomposition process decomposing said digital image representation into a sequence of detail images at multiple successively lower resolution levels and a residual image at a still lower resolution level, wherein each detail image represents an amount of local variation of pixel values within said radiation image at the resolution level of the detail image and wherein a residual image is an approximation of said radiation image with omission of all variations comprised in said detail images, and wherein said decomposition process is such that a reconstruction process exists so that upon application of said reconstruction process to the residual image and the detail images said digital signal representation of the radiation image or a close approximation thereof is obtained, and wherein
(ii) said low resolution version is obtained by applying said reconstruction process to detail images up to an intermediate of said multiple resolution levels.

3. A method according to claim 2 wherein said digital signal representation is decomposed into a pyramidal sequence of said detail images at multiple resolution levels and a residual image.

4. A method according to claim 2 wherein said detail images are modified before being subjected to said reconstruction process.

5. A method according to claim 4 wherein said modification comprises application to at least one of the detail images of a monotonically increasing odd mapping function with a slope that gradually decreases with increasing argument values.

6. A method according to claim 1 wherein said low resolution representation is obtained by subsampling said digital signal representation.

7. A method according to claim 1 wherein said digital image representation is obtained by scanning a photostimulable phosphor screen wherein a radiation image has been stored with stimulating irradiation, detecting light emitted upon stimulation and converting the detected light into a digital signal representation.

8. A method according to claim 1 wherein said radiation images pertain to examinations of a single patient.

9. A method according to claim 1 wherein said recording material is a photographic material.

## Patentansprüche

1. Verfahren zum Reproduzieren von Strahlungsbildern auf einem Aufzeichnungsmaterial, das die folgenden Schritte umfaßt:
(i) Identifizieren von Strahlungsbildern, die reproduziert werden sollen,
(ii) Extrahieren einer reduzierten Version aus einer digitalen Bilddarstellung eines zu reproduzierenden Bilds, wobei die reduzierte Version eine niedrigaufgelöste Version dieses Bilds darstellt, **dadurch gekennzeichnet, daß**
(iii) Bilder gemäß einer Regel identifiziert werden, z.B. Patientenname, Tag oder Radiologieraum,
(iv) für jedes der identifizierten Strahlungsbilder eine entsprechende digitale Bilddarstellung aus einem elektronischen Archiv (12) abgerufen wird,
(v) ein zusammengesetztes Bildsignal, das ein zusammengesetztes Bild darstellt, ausgebildet wird, das für jedes identifizierte Strahlungsbild die niedrigaufgelöste Bildversion umfaßt,
(vi) das zusammengesetzte Bildsignal an ein Ausdruckaufzeichnungsgerät (10) angelegt wird, um auf einem einzelnen Aufzeichnungsmaterial ein zusammengesetztes Bild zu erzeugen, das eine niedrigaufgelöste Version jedes der identifizierten Strahlungsbilder umfaßt.

2. Verfahren nach Anspruch 1, wobei:
(i) ein Strahlungsbild in dem elektronischen Archiv (12) in Form einer Mehrfachauflösungsbilddarstellung gespeichert wird, die erhalten wurde, indem auf die digitale Bilddarstellung des Bilds ein Zerlegungsprozeß angewendet wurde, der die digitale Bilddarstellung in eine Folge von Detailbildern bei mehreren, zunehmend niedrigeren Auflösungsniveaus und ein Restbild bei einem noch niedrigeren Auflösungsniveau zerlegt, wobei jedes Detailbild eine Menge lokaler Schwankungen von Pixelwerten in dem Strahlungsbild bei dem Auflösungsniveau des Detailbilds darstellt und wobei ein Restbild eine Annäherung des Strahlungsbilds unter Auslassung aller in den Detailbildern enthaltenen Schwankungen ist und wobei der Zerlegungsprozeß derart ist, daß ein Rekonstruktionsprozeß existiert, so daß bei seiner Anwendung auf das Restbild und die Detailbilder die digitale Signaldarstellung des Strahlungsbilds oder eine gute Annäherung daran erhalten wird,
(ii) die niedrigaufgelöste Version erhalten wird, indem der Rekonstruktionsprozeß auf Detailbilder bis zu einem Zwischenwert der mehreren Auflösungsniveaus angewendet wird.

3. Verfahren nach Anspruch 2, wobei die digitale Signaldarstellung in eine pyramidenförmige Folge der Detailbilder bei mehreren Auflösungsniveaus und ein Restbild zerlegt wird.

4. Verfahren nach Anspruch 2, wobei die Detailbilder modifiziert werden, bevor sie dem Rekonstruktionsprozeß unterzogen werden.

5. Verfahren nach Anspruch 4, wobei die Modifikation umfaßt, auf mindestens eines der Detailbilder eine monoton zunehmende ungerade Abbildungsfunktion mit einer Neigung anzuwenden, die mit steigenden Argumentwerten allmählich abnimmt.

6. Verfahren nach Anspruch 1, wobei die niedrigaufgelöste Darstellung durch Unterabtastung der digitalen Signaldarstellung erhalten wird.

7. Verfahren nach Anspruch 1, wobei die digitale Bilddarstellung erhalten wird, indem ein fotostimulierbarer Leuchtstoffschirm, in dem ein Strahlungsbild gespeichert worden ist, mit stimulierender Bestrahlung abgetastet wird, das bei Stimulierung emittierte Licht erfaßt und das erfaßte Licht in eine digitale Signaldarstellung umgewandelt wird.

8. Verfahren nach Anspruch 1, wobei die Strahlungsbilder Untersuchungen eines einzelnen Patienten betreffen.

9. Verfahren nach Anspruch 1, wobei das Aufzeichnungsmaterial ein fotografisches Material ist.

## Revendications

1. Procédé de reproduction sur un matériau d'enregistrement d'images obtenues par rayonnement, comprenant les étapes consistant à
(i) identifier des images obtenues par rayonnement destinées à être reproduites,
(ii) extraire d'une représentation d'image numérique d'une image à reproduire une version réduite représentant une version à basse résolution de cette image,
**caractérisé en ce que**
(iii) les images sont identifiées conformément à une règle, par ex. le nom du patient, le jour ou la salle de radiologie,
(iv) pour chacune des images obtenues par rayonnement identifiées, une représentation d'image numérique correspondante est récupérée d'une archive électronique (12),
(v) un signal d'image composite représentant une image composite est formé, comprenant pour chaque image obtenue par rayonnement identifiée ladite version d'image à basse résolution,
(vi) ledit signal d'image composite est appliqué à un enregistreur sur tirage (10) en vue de générer sur un matériau d'enregistrement unique une image composite comprenant une version à basse résolution de chacune des images obtenues par rayonnement identifiées.

2. Procédé selon la revendication 1, dans lequel
(i) une image obtenue par rayonnement a été stockée dans ladite archive électronique (12) sous la forme d'une représentation d'image à résolutions multiples, obtenue en appliquant sur la représentation d'image numérique de ladite image un processus de décomposition décomposant ladite représentation d'image numérique en une suite d'images de détail à des niveaux de résolution multiples de plus en plus bas et en une image résiduelle à un niveau de résolution encore plus bas, où chaque image de détail représente une quantité de variation locale de valeurs de pixel dans ladite image obtenue par rayonnement au niveau de résolution de l'image de détail et où une image résiduelle est une approximation de ladite image obtenue par rayonnement en omettant toutes les variations contenues dans lesdites images de détail, et où ledit processus de décomposition est tel qu'il fait intervenir un processus de reconstruction qui, lorsqu'il est appliqué à l'image résiduelle et aux images de détail, permet d'obtenir ladite représentation de signal numérique de l'image obtenue par rayonnement ou une proche approximation de celle-ci, et où
(ii) ladite version à basse résolution est obtenue en appliquant ledit processus de reconstruction aux images de détail jusqu'à un niveau intermédiaire desdits niveaux de résolution multiples.

3. Procédé selon la revendication 2, dans lequel ladite représentation de signal numérique est décomposée en une suite pyramidale desdites images de détail à des niveaux de résolution multiples et en une image résiduelle.

4. Procédé selon la revendication 2, dans lequel lesdites images de détail sont modifiées avant d'être soumises audit processus de reconstruction.

5. Procédé selon la revendication 4, dans lequel ladite modification comprend l'application à l'une au moins des images de détail d'une fonction de correspondance impaire à croissance monotone dont la pente diminue progressivement avec des valeurs d'argument croissantes.

6. Procédé selon la revendication 1, dans lequel ladite représentation à basse résolution est obtenue par sous-échantillonnage de ladite représentation de signal numérique.

7. Procédé selon la revendication 1, dans lequel ladite représentation d'image numérique est obtenue par balayage d'un écran à luminophore photostimulable dans lequel une image obtenue par rayonnement a été stockée à l'aide d'un rayonnement stimulant, par détection de la lumière émise lors de la stimulation et par conversion de la lumière détectée en une représentation de signal numérique.

8. Procédé selon la revendication 1, dans lequel lesdites images obtenues par rayonnement se réfèrent aux examens d'un même patient.

9. Procédé selon la revendication 1, dans lequel ledit matériau d'enregistrement est un matériau photographique.
